Europäisches Patentamt

European Patent Office

Office européen des brevets

(19)

(11) Publication number: **0 176 365 B1**

(12) **EUROPEAN PATENT SPECIFICATION**

(45) Date of publication of patent specification: 03.04.91

(21) Application number: 85306854.2

(22) Date of filing: 26.09.85

(51) Int. Cl.⁵: **C07K 15/00, C12P 21/00, A61K 39/40, C12N 15/00, C12N 5/00, G01N 33/577, G01N 33/569, //(C12P21/00, C12R1:91)**

The file contains technical information submitted after the application was filed and not included in this specification

(54) **Human monoclonal antibody and its preparation.**

(30) Priority: 26.09.84 JP 201363/84

(43) Date of publication of application:
02.04.86 Bulletin 86/14

(45) Publication of the grant of the patent:
03.04.91 Bulletin 91/14

(84) Designated Contracting States:
AT BE CH DE FR GB IT LI NL SE

(56) References cited:
EP-A- 0 085 747
EP-A- 0 096 839
EP-A- 0 105 804
WO-A-86/01807

CHEMICAL ABSTRACTS, vol. 100, no. 25, 18th
June 1984, page 422, abstract no. 207654v,
Columbus, Ohio, US; D.R. GALLOWAY et al.:
"Production and characterization of mon-
oclonal antibodies to exotoxin A from
Pseudomonass aeruginosa",

(73) Proprietor: SUMITOMO CHEMICAL COMPANY,
LIMITED
Kitahama 4-chome 5-33
Chuo-ku Osaka 541(JP)

Proprietor: SUMITOMO PHARMACEUTICALS
COMPANY, LIMITED
40, Dosho-machi 2-chome
Higashi-ku Osaka-shi Osaka-fu(JP)

(72) Inventor: Noguchi, Hiroshi
4-4-153, Seiwadai
Kawanishi-shi Hyogo 666(JP)
Inventor: Ohtsuka, Hiroshi
16-40-601, Takagihigashimachi
Nishinomiya-shi Hyogo 663(JP)
Inventor: Nakanishi, Tooru
3-8-3-205, Hamakoshien
Nishinomiya-shi Hyogo 663(JP)
Inventor: Nishimoto, Toshiaki
2-14-7, Mefu
Takarazuka-shi Hyogo 665(JP)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Infection and Immunity, Vol. 45, No. 3, pp 604-609 (Sept. 1984)

Infection and Immunity, Vol. 54, No. 1, pp 239-244 (Oct. 1986)

Science, Vol. 199, pp 1439-1444 (31 March 1978)

J. Immunology, Vol. 128, No. 6, pp 2539-2542 (June 1982)

J. Immunology, Vol. 131, no. 3, pp 1201 - 1204 ( Sept 1983 );

"Hybridoma", Vol. 1, No. 3, pp 323 - 328 (1982)

Inventor: **Ochi, Hiroshi**
2-14-7, **Mefu**
**Takarazuka-shi Hyogo 665(JP)**
Inventor: **Kato, Masuhiro**
2-10-2-235, **Sonehigashimadi**
**Toyonaka-shi Osaka 561(JP)**
Inventor: **Ogino, Shigeo**
5-30-202, **Koshikiiwamachi,**
**Nishinomiya-shi Hyogo 662(JP)**

(74) Representative: **Myerscough, Philip Boyd et al**
**J.A.Kemp & Co. 14, South Square Gray's Inn**
**London, WC1R 5EU(GB)**

**Description**

This invention relates to a human monoclonal antibody against an exotoxin of Pseudomonas aeruginosa (hereinafter referred to as Pseudomonas exotoxin) and to its preparation and applications. More particularly, it relates to a therapeutic agent for infections caused by bacteria including Pseudomonas aeruginosa and diagnostic reagents for infections due to exotoxin-producing Pseudomonas aeruginosa, which comprise a human monoclonal antibody against Pseudomonas exotoxin A of Pseudomonas aeruginosa.

With the development of antibiotics, pathogenic bacteria causing infectious diseases have undergone change. That is, the clinical use of antibiotics has been replacing pathogenic bacteria with other types of bacteria.

The bacteria which have in the past been considered to be of low pathogenicity or weak toxicity, in particular Pseudomonas aeruginosa, are now increasingly giving rise to infections. Pseudomonas aeruginosa is now one of the major causes of bacterial infectious diseases.

It is known that infection by Pseudomonas aeruginosa often produces severe symptoms in patients whose immunity is defective or incomplete, such as patients suffering from cancer or burn, or newborn children, and in patients whose immunity is suppressed by the administration of immuno-suppressive drugs. It is known that such infection can even cause the death of such patients. The pathogenic factors of Pseudomonas aeruginosa include its endotoxins, exotoxins and exoenzymes. In particular, the exotoxin is produced by almost all of the clinically isolated strains of Pseudomonas aeruginosa and it is not only cytotoxic but also lethal to various animals.

For the prevention and treatment of bacterial infections, the first choice is chemotherapy using antibiotics and synthetic antibacterial agents. Many antibiotics have been developed, including streptomycin, kanamycin, penicillin and cephalosporin, and these have been presenting marked clinical effects. Most of them show inhibitory activity against almost all Gram-positive cocci represented by Staphylococci, and also against Gram-negative bacteria such as E . coli . However, in spite of much research over a long period and various developments so far, these is only a small number of drugs which are effective against Pseudomonas aeruginosa. Many of these drugs are only bacteriostatically active or inhibit the growth of Pseudomonas aeruginosa. They have no substantial bactericidal activity, and their therapeutic effect is very small when they are applied clinically.

Another example of the limitations of treatment with antibiotics is that antibiotics, which are able to inhibit the growth of bacteria, are not fully effective against the toxins or enzymes which are produced by bacteria and which are responsible for pathogenicity.

It is known to prevent and treat bacterial infections by neutralizing the toxins and enzymes derived from bacteria by the administration of immunoglobulin preparation, that is, by antibody therapy. This method is usually applied in combination with antibiotic therapy and is increasingly replacing antibiotic therapy. Serum with high antibody titer can be obtained by actively immunizing horses, rabbits or other animals, and the antibody treatment administering this serum has been shown in many experiments to have an excellent therapeutic effect on infections induced in various animals. The effectiveness of antibody treatment in which non-human animal serum is administered to human beings is widely acknowledged in the cases of, for example, diphtheria toxin, snake poisoning, etc.

An article by D.R. Galloway et al in "Infection and Immunity", May 1984, p.262-267, reports the production and characterisation of mouse monoclonal antibody to exotoxin A of Pseudomonas aeruginosa . Only one antibody produced proved to be therapeutically active in a mouse experimental Pseudomonas aeruginosa infection test. In any event a survival rate of only 50% was observed (Fig. 5 on p.266). This renders these mouse monoclonal antibodies inherently unsuitable for the treatment or prophylaxis of infection caused by Pseudomonas aeruginosa .

The transfer of foreign protein obtained from non-human animals to the human body is rarely employed in the treatment of bacterial infections, since it often induces severe undesirable side effects such as anaphylaxis and other allergic reactions. There is hence a strong need for the development of human immunoglobulin having a high antibody titer against bacteria, and toxins and enzymes derived from bacteria, and an excellent therapeutic effect against bacterial infections.

EP-A-105804 describes the production of human monoclonal antibodies against tetanus toxin and diphtheria toxin. However, the neutralising activity of such antibodies which is observed in the case of tetanus toxin is lower than the activity achieved using mouse monoclonal antibodies. No quantitative measure of neutralising activity in the case of diphtheria toxin is given.

The human immunoglobulin preparations available hitherto were formulated in intramuscular or intravenous parenteral preparations by collecting blood from healthy people or from patients with a history of bacterial infection, fractionating and purifying the immunoglobulin by known methods, and removing aggregates by the addition of polyethylene glycol, by treatment with a kind of proteinase, by sulfonation, or by DEAE-column chromatography or the like.

These human immunoglobulin preparations have the advantage of being free of side effects such as anaphylaxis, which is observed upon the administration of non-human animal immunoglobulins, but they also have some drawbacks.

First, the antibody titer to bacteria and toxins or enzymes derived from bacteria is low, and a sufficient therapeutic effect may not always be obtained.

Second, it is difficult to procure consistently a large quantity of immunoglobulin having a high antibody titer. It is also extremely difficult to obtain constantly sera with a high titer, and it is likely that the antibody titer may vary in each manufacturing batch, since the blood used as the raw material is collected from many different healthy volunteers or patients.

Third, there is a possibility that HBs virus, or other hepatitis virus, or adult T cell leukemia virus (ATLV, HTLV) might sometimes be present as contaminants in the immunoglobulin preparation.

The present inventors have sought a solution to these problems and have now succeeded in establishing a human cell line continuously producing a human monoclonal antibody effective against infections caused by Pseudomonas aeruginosa . In particular, the present inventors have discovered a human monoclonal antibody against Pseudomonas exotoxin and have established a process for producing a human monoclonal antibody against Pseudomonas exotoxin and reliably and in a large quantity. This process involves human hybrid cells, continuously producing the said monoclonal antibody, being cultured in vitro or in vivo using non-human living organisms such as mice.

Accordingly, the present invention provides synthetic human monoclonal antibody against an exotoxin A of Pseudomonas aeruginosa , of which the exotoxin A neutralising activity is such as to protect mice from death at a 100% survival rate at a dose of 2 µg/head in a mouse experimental Pseudomonas aeruginosa infection test. The invention also provides a pharmaceutical composition comprising a carrier a diluent and a pharmaceutically effective amount of a human monoclonal antibody against exotoxin A of Pseudomonas aeruginosa, of which the exotoxin A neutralising activity is such as to protect mice from death at a 100% survival rate at a dose of 2 µg/head in a mouse experimental Pseudomonas aeruginosa infection test. The synthetic monoclonal antibody and the composition of the invention are useful for the treatment, prevention and diagnosis of infections caused by Pseudomonas aeruginosa . The term "synthetic" as used herein means "produced outside the human body".

In a further aspect, the present invention provides a process for producing a human monoclonal antibody against exotoxin A of Pseudomonas aeruginosa of which the exotoxin A-neutralizing activity is such as to protect mice from death at a 100 % survival rate at a dose of 2 µg/head in a mouse experimental Pseudomonas infection test, which comprises culturing human transformed B lymphocytes which are established by transforming human B lymphocytes producing the said antibody into immortalized cells capable of permanently proliferating and producing the said antibody by infecting the said B cells with Epstein-Barr virus, and recovering the said monoclonal antibody therefrom.

Yet another aspect of the invention is a hybridoma cell line capable of permanently proliferating and producing a human monoclonal antibody against exotoxin A of Pseudomonas aeruginosa of which the exotoxin A-neutralizing activity is such as to protect mice from death at a 100 % survival rate at a dose of 2 µg/head in a mouse experimental Pseudomonas infection test, which is produced by fusing human B lymphocyte sensitized to said exotoxin A which has been transformed to permanently proliferate by Epstein-Barr virus infection, with myeloma cells.

According to the present invention, a human cell line continuously producing a monoclonal antibody specifically reactive with Pseudomonas aeruginosa exotoxin A is prepared. The said cell line may be produced by the following methods:

The first method comprises infecting human lymphocyte B cells (previously sentisized with Pseudomonas aeruginosa or its exotoxin) with Epstein-Barr virus (EB virus) by mixing the cells with the virus in vitro , thereby transforming the B cells into continuously proliferating cells, and selecting the cells producing the desired antibody from the transformants either before or after they have been cloned, thereby establishing the desired cell line which continuously proliferates in vitro and produces the desired antibody.

The second method is carried out as follows:

Human lymphocyte B cells sensitized with Pseudomonas aeruginosa or its exotoxin are fused with myeloma or B lymphoblastoid cells into a hybrid cell line which continuously proliferates in vitro and produces the desired antibody.

The third method is as follows:

The EB virus-transformed cells producing the desired antibody established in the first method are fused with myeloma or B lymphoblastoid cells into a hybrid cell line which continuously proliferates in vitro and

4

produces the desired antibody.

The obtained lymphoblastoid cells are negative against EB virus or other infectious viruses.

The antibody of the present invention is produced in a large quantity by culturing the cell line established as above in vitro or in vivo , e.g., in the abdominal cavity of nude mice (ascites induction), collecting and purifying the antibody secreted in the culture medium or the abdominal ascites.

The human antibody against Pseudomonas aeruginosa exotoxin A provided by the present invention is homogeneous (derived from a monoclone) human antibody which specifically recognizes the antigen determinant of Pseudomonas exotoxin. The human monoclonal antibody of the present invention possesses either an ability to bind to Pseudomonas exotoxin (binding ability), or an ability to inhibit invasion of the exotoxin into cells, or an ability to inhibit the enzymatic action of the exotoxin (neutralizing activity), and can be used for the treatment of infections caused by Pseudomonas aeruginosa producing the exotoxin.

Furthermore, it may be used for isolation and identification of exotoxin-producing Pseudomonas aeruginosa .

This invention will be further described below.

The Pseudomonas exotoxin as an antigen refers to a toxin produced by Pseudomonas aeruginosa , being represented by Exotoxin A [M.L. Vasil, D. Kabat and B.H. Iglewski: Infection and Immunity, 16 , 353 (1977); and G.L. Gray et al.: Proc. Natl. Acad. Sci. USA, 81 , 2645 (1984)].

The manufacturing method of the human monoclonal antibody of this invention may be basically divided into the following steps: (1) preparation of human lymphocyte B cells sensitized with an antigen of interest, (2) establishment of a cell line producing a particular monoclonal antibody by conferring to the B cells an ability to permanently proliferate; (3) cultivation of the cell line; (4) collection and purification of the antibody from the culture medium; and (5) preparation of an immunoglobulin preparation with a high antibody titer which contains the monoclonal antibody. These steps are described in more detail below:

The sensitized human lymphocyte B cells used in this process are human lymphatic cells which produce antibodies to Pseudomonas exotoxin, and are mainly isolated by the centrifugal method from the peripheral blood by using lymphocyte separating agents such as Lymphoprep®, Nyegaard & Co., Oslo, Norway and Mono-Poly Resolving Medium®, Flow Laboratories Ltd., McLean, Virginia,U.S.A. Alternatively, lymphocyte B cells derived from lymph nodes, spleen or other organs removed for the purposes of diagnosis or treatments of diseases or from the cord blood may be used in preparing the sensitized lymphocyte B cells. It is preferable to use sensitized human lymphocyte B cells derived from patients having a history of infection by Pseudomonas aeruginosa , in particular, infection by exotoxin-producing Pseudomonas aeruginosa . By measuring the antibody titer in the serum, proper donors of the lymphocytes may be selected. One other method is to collect lymphocyte B cells from a donor, regardless of whether or not the donor has a history of infection by Pseudomonas aeruginosa , and sensitize the cells in vitro by mixing them with inactivated Pseudomonas exotoxin. That is, the inactivated Pseudomonas exotoxin (antigen) is added to lymphocyte B cells. Subsequently, vegetable lectin such as pokeweed mitogen (PWM) lectin, Staphylococcus aureus Cowan I and other bacterial component, human lymphocyte-mixed culture media, spleen, thymus or cord blood cell culture media, or solution containing lymphokines such as B cell proliferating factors and B cell differentiating factors are added to the cells either simultaneously or in various combinations so that they are sensitized with antigen in vitro and successively proliferated and differentiated into antigen-producing cells. These human lymphocyte B cells all possess antibody molecules on their cell surface and can secrete a small quantity of antibodies for a limited period, but cannot be proliferated permanently.

This invention includes two methods for obtaining cell lines that can limitlessly and continuously proliferate from the human lymphocyte B cells which are sensitized with the antigen. In the first method, human lymphocyte B cells sensitized with antigen are cultured with EB virus derived from marmoset cells B95-8 so that the lymphocyte B cells are infected with the EB virus. Preferably, 2 to 10 $TD_{50}$ viruses are mixed per cell. 0.5 to 3 x $10^4$ cells per well are seeded in a 96-well microplate, and cultured for 2- or 5-weeks at 32 to 37°C in the presence of 5 to 10% of $CO_2$ in a conventional medium such as RPMI 1640 or Eagle's MEM containing 2 to 20% (v/v) of preselected serum such as fetal bovine, bovine, equine or human (preferably fetal bovine) serum.

During cultivation, the medium is replaced by a half portion every two to four days. As required, antibiotics or synthetic antibacterial compounds are added to prevent contamination with mycoplasma.

The transformed cells are, after 10 days of infection, observed under an optical microscope in a cluster of 20 to 200 cells, and are easily distinguished from nontransformed cells. The antibody titer of the culture medium in each well containing sufficiently grown transformant cells is assayed by the enzyme-linked immunosorbent assay (ELISA) to select wells containing cells producing the antibody.

The selected transformant cells are then cloned by the so-called limiting dilution method. For example, the solution containing the transformant cell cluster is lightly sucked and discharged repeatedly with a pipette so as to loosen the cluster and diluted to a proper cell density with a culture medium, which is then seeded in a 96-well microplate at about 0.5 to 100 cells/well and cultivated. Alternatively, they may be cloned by using soft agar. For example, about $7 \times 10^4$ to $7 \times 10^5$ transformed cells are mixed in 2 ml of 0.36 to 0.4% (w/v) soft agar (preferably sea plaque agarose), and the soft agar is overlayered onto the solidified agar layer consisting of 3 ml of 0.5% agar in culture plate (30 mm in diameter) to be cultivated at 5% $CO_2$, $37°C$. Thus, colonies growing from one cell are obtained. At the time of cloning, it is preferable to use cells which are present in the abdominal cavity of mouse, human cord blood lymphocytes or X-ray irradiated spleen cells of mouse, as the feeder layer. The antibody titer of the culture supernatant of the cloned cell lines is measured by the ELISA method to further select the cell line with the greatest production of specific antibodies. By repeating this cloning and selection of the cells with a high antibody production two or three times, transformed cell lines which grow rapidly and which are capable of producing the monoclonal antibody stably and in a large quantity are established.

In the second method, human lymphocyte B cells sensitized with antigen and myeloma cells are fused in the presence of polyethylene glycol (PEG) . The myeloma cells used are hypoxanthine-guanine-phosphoribosyltransferase(HGPRT)-deficient mutants derived from mouse myeloma cells, such as P3X63-Ag8Ul(P3Ul) provided by Dr. Maruyama, Osaka Univ., Osaka and Dr. Watanabe, Saga Medical College, Saga; ref. Yelton D.E., et al.: Curr. Top. Microbial. Immunol.81 , 1, 1978 and P3X63-Ag8.653 provided by Dr. Watanabe, Saga Medical College, Saga; Human Genetic Mutant Cell Repository, Camden, N.J.; ref. Kearney J.F., et al.: J. Immunol. 123 , 1548, 1979, or HGPRT-deficient mutant derived from human myeloma cell (Sikora K. and Neville A.M., Nature 300 , 316, 1982; Lane, H.C. and Fauci, A.S., In Monoclonal antibodies (ed. by Haynes and Eisenbarth), p.131, 1983 Academic Press, N.Y.). HGPRT-deficient mutant derived from human lymphoblastoid cells(Human Genetic Mutant Cell Repository Camden, N.J.)may also be used in place of the myeloma cells. In this method, PEG 1,000 - 6,000 is used at a concentration of 30 to 50% (w/v). The efficiency of fusion may be enhanced by adding lectin, poly-L-lysine or DMSO. HVJ virus may be used in place of PEG. The fusion is conducted according to the method proposed by Köhler and Milstein (Nature 256 , 495, 1975) wherein mouse cells are fused to produce hybridoma-producing mouse monoclonal antibodies. More specifically, human lymphocyte B cells sensitized with antigen are mixed with HGPRT-deficient myeloma cells in a ratio of from 10:1 to 1:1, and 30 to 50% (w/v) of PEG 6,000 is added portionwise over a period of 0.5 to 1 minute, and the solution is allowed to stand for 1 to 10 minutes. Thereafter, 10 to 50 ml serum-free medium is added over a period of 5 to 10 minutes. Additional medium is added to adjust the cell density to $10^5$ to $10^6$ cells/ml, and $2 \times 10^4$ to $2 \times 10^5$ cells/well are seeded in a 96-well microplate. The next day, half of the medium is replaced by hypoxanthine-aminopterin thymidine-containing medium (HAT medium), and is cultivated at 5% $CO_2$ at 32 to $37°C$. Thereafter, the cells are cultured in fresh HAT medium for about 10 to 20 days, and in fresh hypoxanthine-thymidine containing medium (HT medium) for the following about 3 to 5 days, while half of the medium is replaced with the above mentioned fresh media once every three days. After the cultivation has been performed for a total of 2 to 3 weeks after the fusion, the proliferating colonies, or so-called hybridomas, are obtained. Without using HGPRT-deficient mutant, it is also possible to select the hybridoma by using antimetabolites such as actinomycin D and emetine. The cell line producing a monospecific antibody against Pseudomonas exotoxin is selected by measuring the antibody titer of the culture medium of the hybridoma cells by ELISA or radioimmunoassay (RIA). By the limiting dilution method or soft agar method, cloning may be carried out or 3 times to obtain cell lines which are fast-growing, stable and able to produce a large quantity of the monospecific antibody. The cells transformed by EB virus in the first method may also be used in place of the human lymphocyte B cells sensitized with the antigen. In this case, it is preferable to mix and fuse the EB virus-transformed cells with HGPRT-deficient myeloma in a ratio of 5:1 to 0.2 : 1.

The cell lines established with the antigen-sensitized human lymphocyte B cells by the EB virus transformation method or cell fusion method (hybridoma method) are characterized by its continuous proliferation and its stable and abundant production of the monospecific antibody.

Thus established transformed cells or hybridomas ($0.5 \times 10^5$ to $5 \times 10^5$ cells/ml) are cultured by stationary or rotary cultivation in an adequate vessel such as a culture flask or plate with a medium usually used for the cultivation of animal cells under the conditions of 2 to 10% $CO_2$, at 32 to $37°C$ in a $CO_2$ incubator. When cultivating in a large quantity, a jar fermenter or hollowfiber system designed for animal cells may be used. There may be used such media as RPMI1640 or Eagle's MEM etc.containing 2 to 20 % of fetal bovine, calf, bovine, equine or human serum, or serum-free medium containing traces of components necessary for cell proliferation, such as insulin, transferin, ethanolamine, selenite, bovine albumin and lipid. Besides the in vitro cultivation mentioned above, it is also possible to cultivate the cells in the abdominal cavity or other part of the body of animals such as nude mice by inoculating the transformed cells or hybridomas into it. Usually, $0.5 \times 10^7$ to $2.5 \times 10^7$ cells are intraperitoneally administered per mouse or nude mouse. In this case, before inoculation of cells, it is preferably to administer pristane or antiasialo-$GM_1$ antibody. X-ray irradiation or splenectomy is sometimes effective, too.

The antibody of the present invention may be purified by conventional biochemical techniques. The following well-known methods may be used, alone or in combination; ammonium sulfate precipitation fractionation, ethanol precipitation fractionation, PEG fractionation, ion exchange chromatography, gel filtration, affinity chromatography, high performance liquid chromatography, and electrophoresis. In the purification process, it is necessary to prevent the formation of aggregate and the reduction of antibody activity. Human serum albumin (HSA) is suitably added to the antibody preparation at a concentration of 0.05 to 2% for this purpose. However, it may sometimes be preferable to add, instead, glycine, $\alpha$-alanine or other amino acids, especially lysine, arginine, histidine or other basic amino acids, glucose, mannitol or other sugars, and sodium chloride or other salts. In the case of IgM antibody, which is known to aggregate easily, treatment with $\beta$-propionolactone or acetic anhydride may prevent aggregation and make intravenous injection possible.

The purified human monoclonal antibody may be made into pharmaceutical preparations by the usual preparation method for biological products. Basically, after filtration and sterilization by a membrane filter or the like, it is freeze-dried together with stabilizers and packed in a sterile vial.

The present human monoclonal antibody preparation comprises at least a human monoclonal antibody against exotoxin A of Pseudomonas aeruginosa as the agent for treating and preventing bacterial infection. However, it preferably includes two or more human monoclonal antibodies capable of recognizing different antigenic determinants of the Pseudomonas exotoxin molecule. Furthermore, it may be used in combination with a conventional human antibody capable of recognizing an antigen derived from Pseudomonas aeruginosa other than an exotoxin A, for example, a Pseudomonas exoenzyme such as elastase or protease, toxin constituents, or outer membrane protein. Furthermore, conventional human antibodies against bacteria other than Pseudomonas aeruginosa , virus, fungi, protozoa or cancer cells may be used together with the human monoclonal antibody against Pseudomonas aeruginosa exotoxin A obtained in this invention. By adding the human monoclonal antibody obtained according to this invention to a conventional human immunoglobulin preparation, a high titer immunoglobulin preparation against the exotoxin may be prepared.

The human monoclonal antibody obtained according to this invention usually belongs to class IgG or IgM, but is not limited to these. The present human monoclonal antibody binds to Pseudomonas exotoxin (binding activity). It is desirable for the antibody to have a high binding activity of $10^8$ liter/mol or higher. It may also have the ability to inhibit the invasion of exotoxin into cells, and the ability to inhibit enzymatic activity of exotoxin (neutralizing activity). Mice experimentally infected with exotoxin-producing Pseudomonas aeruginosa are protected from death at a 100% survival rate at a dose of 2 $\mu$g/head of the antibody of the present invention.

When used for the treatment or prevention of infection with exotoxin-producing Pseudomonas aeruginosa , or mixed bacterial infection including this microorganism, the human immunoglobulin preparation containing at least one of the human monoclonal antibodies of the present invention is administered at a dose rate of from 1 to 10 g in adult patients with severe symptoms, or from 0.2 to 5 g in milder cases.

As described above, the human monoclonal antibody against an exotoxin A of Pseudomonas aeruginosa obtained according to this invention possesses a high antibody titer against the antigen, and exhibits a far better therapeutic activity than conventional immunoglobulin preparations in mouse experimental infection tests. Moreover, since it is a protein derived from human, the monoclonal antibody is expected to cause only low levels of side effects such as anaphylaxis, which is experienced by subjects treated with foreign protein. Also, since the antibody is produced and purified from specific cells, it is unlikely to be contaminated with other components of unknown biohazard, which is a problem with conventional immunoglobulin prepared from the blood of many people. The characteristics of the method for production of the monoclonal antibody are, firstly, that the specific antibody with a high antibody titer is consistently and reliably produced in vitro in a large quantity. Secondly, the method is superior to conventional methods in which the product is prepared from a human blood pool because the present method produces a higher antibody titer and affords quality control which makes it possible to supply the product stably.

The invention will be further described and illustrated in the following examples, but is no limited thereto.

EXAMPLE 1

Establishment of human monoclonal antibody-producing cell line by EB virus transformation method.

(I) Preparation of EB virus solution and assay of its viral titer.

Marmoset B95-8 cells (provided by Dr. Yamane, Tohoku Univ. Sendai; Human Genetic Mutant Cell Repository, Camden, N.J.) producing EB virus were suspended at a density of $6.5 \times 10^5$ cells/ml in RPMI 1640 medium containing 10% (v/v) of fetal calf serum (FCS) and were cultivated for 4 days at 5% $CO_2$, 37°C, by using a culture flask T-75 (Corning Glass Works, Corning, N.Y. #25110). The supernatant of the culture medium was collected, and was centrifuged for 10 minutes by a low speed centrifuge (Tomy Seiko Co., Ltd. Tokyo, Japan RS-20BH) at 2,000 rpm (rotor TS-7), and its supernatant was filtered by 0.45μ membrane filter Myrex SLHA 02505 (Millipore Corporation, Bedford, MA). This filtrate was distributed into serum tubes (Sumitomo Bakelite Company Ltd. Tokyo, Japan MS-4505) and stored at -80°C, and was dissolved when necessary and used in the experiment of transformation of human lymphocytes with EB virus. The viral titer of the EB virus solution was measured by the method proposed by D.J. Moss and J.H. Pope (J. General Virology, 17, 233, 1972), using the human peripheral blood lymphocyte as indicator cells. That is, a suspension of human peripheral blood lymphocytes ($10^6$ cells/ml) in the RPMI1640 containing 16% of FCS was distributed into a 96-well microplate (Sumitomo Bakelite Co. Ltd.) in an amount of 100 μl per well. Then, each 20 μl of the EB virus solution serially diluted 10-fold with the same medium in the range of $10^0$ to $10^7$ was added thereto, and cultivated at 5% $CO_2$, 37°C.

The cells were grown for 3 weeks while half of the culture medium was replaced with fresh medium at 3 day intervals and the presence of the transformed cells was investigated by observing with an optical microscope. Transformation rate was determined using six wells at every step of dilution. The maximum dilution rate required to transform 50% of the cells was statistically determined by the Reed and Muench method (Reed, J.J. & Muench, H., American J. of Hygiene, 27, 493-497, 1938)from the transformation rate of each dilution step, the viral quantity in it was taken as $\overline{1}$ $TD_{50}$, and the viral quantity in the original sample was inversely calculated to obtain $TD_{50}$. An EB virus solution with viral quantity of $10^5$ to $10^7$ $TD_{50}$/ml was obtained.

(2) Measurement of titer of anti-Pseudomonas exotoxin antibody by ELISA.

The titer of anti-Pseudomonas exotoxin antibody was measured by the method proposed by S. J. Cryz, E. Fürer, R. Germainer (Infection and Immunity, 40, 659, 1983). That is, the Pseudomonas exotoxin (provided by Dr. Homma, Kitasato Inst., Tokyo and bought from Swiss Serum and Vaccine Institute Berne, Berne, Switzerland)solution diluted to a concentration of 5 μg/ml in 0.1 M sodium bicarbonate buffer solution (pH 9.6) was distributed by 100 μl per well into a 96-well microplate (Falcon, Division of Becton-Dickinson and Company, Oxnard, CA #3912, hereinafter called microplate), and was incubated for 2 hours at 37°C, whereby the exotoxin was adsorbed on the microplate.

After the exotoxin solution was removed from the microplate, the phosphate buffered saline solution (called PBS) pH 7.2 [composition: NaCl (8 g/liter), KCl (0.2 g/liter), $Na_2HPO_4 \cdot 12H_2O$ (2.9 g/ liter) and $KH_2PO_4$ (0.2 g/liter)] containing 3% of bovine serum albumin (BSA) was distributed at 120 μl per well, and incubated for 30 minutes at 37°C in order to block the exotoxin-unadsorbed portion. The microplates were used in the subsequent operation as an antigen-adsorbed plate. It was stored at -20°C, as required, at this stage. Before the assay, the antigen-adsorbed plate was washed three times with PBS containing 0.05% of Tween® 20 (Polyoxyethylene Sorbitan Monolaurate; Nakarai Chemicals Ltd., Kyoto, Japan) (called PBST). The PBST containing 1% of BSA was distributed at 50 μl per well, and the sample (serum ascites or culture supernatant) diluted in PBST containing 1% of BSA was added at 50 μl per well, and incubated for 2 hours at 37°C. Then the sample solution was removed, and the microplate was washed three times with PBST. A solution of the second antibody defined below was added at 100 μl per well, and incubated for 2 hours at 37°C. As the second antibody, phosphatase-labeled and affinity purified anti-human immunogloblin antibody (Kirkegaard & Perry Lab. Inc. Gaithersburg, MD) was used after diluting 500 to 1,000 times with PBS containing 1% of BSA. For the measurement of IgG and IgM antibody titers, phosphatase labeled anti-human IgG and IgM antibodies were used, respectively. After the second antibody solution was removed, the microplate was washed three times with PBST. A coloring substrate solution (an aqueous solution having 3 mg of p-nitrophenyl disodium phosphate dissolved in 1 ml of 10% diethanolamine buffer solution at pH 9.1 containing $NaN_3$ (0.2 mg/ml) and $MgCl_2 \cdot 6H_2O$ (0.1 mg/ml)) was added at 100 μl per well and a reaction performed at 37°C After 45 minutes 3 N NaOH was added at 20 μl per well to stop the reaction. After the reaction was over, the $OD_{405}$ was measured by multiscanner (Titertek® Multiskan MCC, Flow Laboratories, Inc. McLean, Virginia).

(3) Selection of lymphocyte donors.

Peripheral blood was collected from 29 human volunteers, and the antibody titer of the anti-Pseudomonas exotoxin of the isolated serum sample was measured by the ELISA method. The results are partly shown in Table 1. Five samples showed high titer of anti-exotoxin IgG antibody, and one of them showed relatively higher anti-exotoxin IgM antibody titer.

Table 1. Titer of Anti-Pseudomonas Exotoxin Antibody
in Human Serum Samples

| Serum | Antibody titer ($OD_{405}$) | |
|---|---|---|
| | IgG | IgM |
| No. 1 | >2.0 | 0.2 |
| No. 2 | 0.1 | 0.2 |
| No. 3 | 2.0 | 0.3 |
| No. 4 | 0.1 | 0.1 |
| No. 5 | 1.8 | 0.1 |
| No. 6 | 1.6 | 0.1 |
| No. 7 | >2.0 | 0.1 |
| No. 8 | 0.4 | 0.1 |
| No. 9 | 0.2 | 0.1 |
| No.10 | 0.4 | 0.1 |
| Control (Negative) | $0.5\pm0.3$ | $0.1\pm0.1$ |

(4) Preparation of lymphocytes from human peripheral blood.

Peripheral blood (100ml)was taken from human volunteer No. 1 showing a high titer of anti-Pseudomonas exotoxin antibody in serum. 15 ml of Mono-Poly Resolving Medium® (Flow Laboratories Inc., McLean, Virginia) was put in a centrifugal tube (Sumitomo Bakelite Company Ltd., Tokyo Japan, 50 ml capacity), and 20ml of the peripheral blood was slowly overlaid on it. The solution was centrifuged in a low speed centrifuge (Tomy Seiko Co. Ltd., Tokyo, Japan, RS-20BH) for 15 minutes at room temperature at a speed of 2,500 rpm (rotor TS-7), causing erythrocytes to be separated from lymphocytes. The portion containing lymphocytes was recovered and washed twice with RPMI 1640 medium containing 10% of FCS (hereinafter referred to as the medium) , and the number of cells was counted. $1.2 \times 10^8$ lymphocytes were obtained.

(5) Establishment of anti-Pseudomonas exotoxin antibody-producing cell line by EB virus transformation method.

To a suspension of $2 \times 10^7$ cells of peripheral blood lymphocytes of human volunteer No. 1 in 2 ml of the medium, was added 10 ml of said EB virus solution (with viral quantity of $10^7$ $TD_{50}$/ml). The solution was incubated for 2 hours at 5% $CO_2$ at 37°C and then centrifuged in a low speed centrifuge (Tomy Seiko RS-20BH) for 2 minutes at 10°C, at 2,000 rpm (rotor TS-7) to recover the human lymphocytes infected with EB virus. The EB virus-infected lymphocytes were suspended in a medium containing 10% of fetal bovine serum, penicillin (100 IU/ml), streptomycin (50 $\mu$g/ ml), polymyxin (25 $\mu$g/ml), spectinomycin (20 $\mu$g/ml), and arginine (0.2 mg/ml) (Microplasma Cocktail, Bradley et al., 5th International Congress of Immunology, Kyoto, 1983, abbreviated as MPC) (about $2 \times 10^5$ cells/ ml), and 100 $\mu$l per well of the suspension was seeded in microplates to which a suspension of mouse peritoneal cells ($3 \times 10^4$ cells/well) had previously been added. After culturing for one week, half the medium was replaced with fresh MPC every three days, and the growth of cells was observed three weeks later. In all the wells, the growth of EB virus-transformed cells was observed. Furthermore, the titer of the anti-exotoxin antibody contained in the supernatant of the culture medium was measured by ELISA method. Wells with a high titer of anti-exotoxin IgG antibody were noted at a rate of 1/960 (FK-5A5). In the supernatant, human IgG was contained in an amount of 2 $\mu$g/ml, and its antibody titer was the same as that of serum No. 1 diluted 50 times.

(6) Cloning.

Cells from the well with a high titer of anti-exotoxin antibody were suspended in the MPC at $10^3$ cells/ml, which was seeded in an amount of 100 $\mu$l per well in a microplate to which a suspension of mouse peritoneal cells ($3 \times 10^4$ cells/well) had previously been added. After cultivating for 5 days, one-half of the medium was replaced with fresh MPC every three days. Three weeks later, the titer of the anti-exotoxin antibody contained in the culture supernatant was measured. After cultivating for 3 weeks as above, growth of transformed cells was observed in 68 out of 96 wells. Of these the anti-exotoxin antibody titer was high in 3 wells, and relatively high in 6 wells. Similarly, using cells in the wells with high antibody titer, we established a cell line FK-5A5 which produces anti-exotoxin antibodies stably.

## Example 2

Establishment of human monoclonal antibody-producing cell line by EB virus transformation.

Preparation of EB virus solution, assay by ELISA, and preparation of human peripheral blood lymphocytes were carried out in the same manner as in Example 1. Establishment of anti-Pseudomonas exotoxin antibody-producing cell line by EB virus transformation was effected basically in the same manner as in Example 1. That is, $9.0 \times 10^6$ lymphocytes of peripheral blood of human volunteer No. 1 were mixed with 5 ml of viral solution having viral infectivity of $10^7$ $TD_{50}$/ml to infect the lymphocytes. The cells were seeded in microplates in an amount of $1.8 \times 10^4$ cells per well, and cultivated as in Example 1. After 12 days of cultivation, growth of cells was observed in all wells, and wells with a high titer of anti-Pseudomonas exotoxin antibody were obtained at a rate of 1/480. The obtained cell line FK-001 was able to produce anti-Pseudomonas exotoxin antibody stably without lowering the antibody titer. The class of the antibody produced was IgM.

The lymphoblastoid cell FK-001 thus obtained was confirmed to be negative against viral capsid antigen (VCA) of EB virus by immunological method with KAYAKU VCA kit (Wako-Pure Chemicals, Tokyo, Japan). It indicates that lymphoblastoid cell FK-001 does not produce any EB virus particle.

## Example 3

Establishment of human monoclonal anti-Pseudomonas exotoxin antibody-producing cell line by human-mouse cell fusion.

(1) Measurement of anti-Pseudomonas exotoxin antibody titer by radioimmunoassay (RIA) method.

A 96-well microplate (Falcon #3912) was washed, then 200 $\mu$l per well of PBS containing 3% of BSA was added thereto and incubated for 30 minutes at 37°C. After removal of the PBS containing 3% of BSA, the microplate was dried at 37°C for 10 minutes and then washed three times with PBS. Thereafter, 50 $\mu$l of supernatant of a culture medium or ascites diluted in PBS containing 1% of BSA, and 50 $\mu$l of $^{125}$I-labeled Pseudomonal exotoxin (about 20,000 cpm specific activity 18 mCi/mg) in turn diluted in PBS containing 1% of BSA were added to each well, and incubated at 37°C for 2 hours. The microplate was allowed to stand overnight at 4°C. The next day, 50 $\mu$l of rabbit anti-human IgM (G) antiserum (MILES-YEDA Ltd., Rehovot, Israel, #65-066 anti-IgG, #65-067 anti-IgM) diluted in PBS was added to each well, and was stirred for an hour at 37°C. Twenty $\mu$l of Protein A-Sepharose (Pharmacia, Uppsala, Sweden) suspended in PBS containing 1 mg/ml of gelatin and 0.02% of $NaN_3$ was added to each well, and stirred for an hour at 4°C. The microplate was centrifuged for 10 minutes at 1,500 x g. After centrifuging, half of the supernatant was removed, and, instead, Tris-hydrochloric acid buffer (50 mM, pH 7.5, hereinafter called washing buffer) containing 5 mM EDTA, 1 mg/ml gelatin, 0.02% $NaN_3$, and 150 mM NaCl was added to each well. After centrifuging again, half of the supernatant was replaced by the washing buffer. After repeating this operation three to five times, each well was separated, and the radio-activity was measured by a $\gamma$-counter.

(2) Preparation of human peripheral blood lymphocytes sensitized with antigen.

Lymphocyte B cells were collected from the human peripheral blood using the same procedure described in Example 1-(3) and (4).

(3) Cell fusion.

Mouse BALB/C myeloma cells P3X63-Ag8U1 (referred to as P3U1) (Marguilies et al., Cold Spring Harbor Symp. Quant. Biol., 41 , 781, 1976) derived from MOPC-21 and deficient in HGPRT (Hypoxanthine guanine phosphoribosyltransferase), were cultivated in passages in the RPMI1640 medium (containing 10% of fetal bovine serum) or MPC, and $10^7$ cells thereof were washed three times with Eagle's MEM. $1 \times 10^7$ to $5 \times 10^7$ of lymphocytes of human peripheral blood, which were isolated using Lymphoprep® (Nyegaard & Co., Oslo, Norway), instead of the Mono-Poly Resolving Medium® used in Example 1-(4), were washed three times with Eagle's MEM, mixed with $10^7$ of the myeloma cells in a centrifugal tube (Corning #25330), and centrifuged for 7 minutes at 400 x g to give pellets. To the pellets, 1 ml of PEG #6,000 (45% Eagle's MEM solution, KOCH-LIGHT Laboratories Ltd., Berks, England) was added over 30 seconds, while the centrifugal tube was rotated. The mixture was allowed to stand for 3 minutes. Then Eagle's MEM was added at a rate of 2 ml per minute while the centrifugal tube was rotated, and this operation was repeated seven times. Finally about 20 ml of Eagle's MEM was added, and the mixture was centrifuged for 7 minutes at 400 x g. After centrifuging, the pellets were suspended in 150 ml of RPMI1640 medium containing 15% of fetal bovine serum, 2 mM L-glutamine, and 1 mM sodium pyruvate or MPC, which was distributed in a 96-well microplate (Falcon #3040) at a final density of 6.7 x $10^4$ myeloma cells per well. At the same time as the feeder layer, mouse BALB/C spleen cells were added to each well at a final density of 5 x $10^5$/ml. This microplate was incubated at 5% $CO_2$, 37°C, and half of the medium was replaced by HAT selective medium every two or three days starting from the next day. About two weeks after the fusion, in the supernatant of the well in which cell growth was confirmed, anti-Pseudomonas exotoxin antibody production was investigated by ELISA [Example 1-(2)] or RIA [Example 3-(1)]. some of the results are shown in Table 2. Hybridomas in the well showing a relatively high antibody titer were increased by cultivation and were also cloned by limiting dilution. About two weeks later, the anti-Pseudomonas exotoxin antibody production in the supernatant of the culture medium of the clone was similarly investigated, and four types of clone were finally obtained.

### Table 2. Anti-Pseudomonas Exotoxin Antibody Titer in Hybridoma Culture Supernatant

#### (1) Before cloning

| Well | Antibody titer (cpm) | Well | Antibody titer (cpm) |
|---|---|---|---|
| 1A11 | 540 | 2 H6 | 1,056 |
| 1B4 | 516 | 3 B10 | 661 |
| 1E6 | 1,014 | 3 F6 | 1,000 |
| 1E9 | 927 | 3 F8 | 824 |

| 1F9 | 2,428 | | 3 G4 | 594 |
|-----|-------|---|------|-----|
| 1G12 | 550 | | 3 G8 | 518 |
| 2B8 | 540 | | 4 B1 | 1,506 |
| 2D1 | 410 | | 4 G4 | 957 |
| 2G10 | 506 | | 4 G6 | 1,405 |

(2)　After cloning

| Clone | Antibody titer (cpm) |
|-------|----------------------|
| 1F9-1E3 | 714 |
| 1H2 | 991 |
| 2A4 | 698 |
| 2A12 | 796 |
| 2B4 | 1,062 |
| 3G8-2A9 | 690 |
| 3E11 | 675 |
| 3F11 | 831 |
| 4G6-1C10 | 769 |
| 1C12 | 1,767 |

The antibody titer was measured by RIA (see Example 3-(1)).

(4) Cultivation of the hybridoma cells, preparation of antibodies, and measurement of antibody titer.

The thus obtained cloned hybridoma and the hybridoma in the antibody producing well before cloning were cultured in vitro , and the supernatants of the media were obtained. About $10^7$ hybridomas were injected into the abdominal cavity of nude mouse (Nihhon Clea, Tokyo) which was pretreated with pristane (2,6,10,14-tetramethylpentadecane) and cultivated in vivo . Ascites were collected to measure the antibody titer by RIA (see Example 3-(1)). Some of the results are shown in Table 3. The ascites growing hybridoma 3F6 showed a significantly higher antibody titer than the ascites of myeloma cell P3U1 used as the control.

Table 3.　Anti-Pseudomonas Exotoxin Antibody Titer
of Ascites Derived from Hydridoma

| Antibody dilution | Antibody titer (cpm) | | |
|-------------------|------|------|------|
| | 3F6 | 1E6 | P3U1 (control) |
| 1:3 | 2,428 | 603 | 627 |
| 1:18 | 2,274 | 960 | 750 |
| 1:108 | 1,848 | 795 | 877 |
| 1:648 | 1,292 | 614 | – |

The antibody titer was measured by RIA (see Example 3-(1)).

(5) Specificity of antibody.

It was confirmed by the competitive inhibition experiment using the RIA (see Example 3-(1)) that the antibody in the ascites of hybridoma cells reacts specifically with the Pseudomonas exotoxin. As mentioned in Example 3-(1) a 96-well microplate, (Falcon #3912) to which BSA had preliminarily been adsorbed, was washed and then 50 $\mu$l of ascites diluted in PBS containing 1% of BSA, 50 $\mu$l of a solution of $^{125}$I-labeled Pseudomonas exotoxin (about 20,000 cpm) diluted in PBS containing 1% of BSA and 10 $\mu$l of a solution of unlabeled Pseudomonas exotoxin diluted in PBS containing 1% of BSA at various concentrations were added to each well. Incubation took place for 2 hours at 37°C, and then the microplate was allowed to stand overnight at 4°C. Rabbit anti-human IgM (G) antiserum and Protein A-Sepharose (Pharmacia, Uppsala, Sweden) were successively added, incubated, centrifuged and washed, and the radioactivity of the antigen antibody complex was measured as explained in Example 3-(1). The results with the antibody in ascites of hybridoma 3F6 are shown in Table 4. The competitive inhibition was carried out upon the simultaneous addition of labeled and unlabeled antigens. It was thus confirmed that the present antibody reacts specifically with the Pseudomonas exotoxin.

Table 4.  Specificity of Antibody in Ascites Derived from Hybridoma 3F6

| Exotoxin amount as competitor (µg) | Antibody titer (cpm) |
|---|---|
| 0 | 2,310 |
| 0.1 | 931 |
| 1 | 853 |
| 10 | 700 |

The background value was 650 to 750 cpm.

Example 4

Establishment of human monoclonal anti-Pseudomonas exotoxin antibody-producing cell line by human-human cell fusion.

Human lymphoblastoid cells NC0467.3 (provided by Dr. Rosen, Karolinska Inst., Stockholm; ref. Chiorazzi et al., J. Exp. Med. 156 , 930, 1982) derived·from PGLC33H line and deficient in HGPRT (hypoxanthine guanine phosphoribosyltransfase) were cultured in passage in the RPMI1640 medium which contains 10% fetal bovine serum. $10^7$ cells thereof were fused with 1 x $10^7$ to 5 x $10^7$ cells of human peripheral blood lymphocytes in the same manner as in preparation of hybridoma in Example 3. About three weeks after the fusion, the supernatant of the well of growing cells was examined for the anti-Pseudomonas exotoxin antibody production according to the ELISA (Example 1-(2)) or RIA (Example 3-(1)), and the hybridoma in the antibody-producing well was cloned by limiting dilution. About three weeks later, the supernatant of the culture medium of the clone was similarly inspected for anti-Pseudomonas exotoxin antibody production in the same manner, and antibody-producing clones were obtained.

Example 5

Cell fusion by using EB virus transformed cells.

0.5 x $10^7$ cells of human lymphoblastoid cells FK-001, which were transformed by EB virus (prepared from Marmoset B95-8 cells as described in Example 1-(1)) in the same manner as in Example 2 and which produce specific antibody against Pseudomonal exotoxin, were fused with 2.5 x $10^7$ cells of mouse myeloma P3·NS-1·1-Ag4-1 (so called NS-1 provided by Dr. Watanabe, Saga Medical College, Saga; Human Genetic Mutant Cell Repository, Camden, N.J.; ref. Köhler, G. and Milstein, C.: Eur. J. Immunol. 6 , 511, 1976) in the same manner as in the preparation of human-mouse hybridoma (Example 3). The fused cells were suspended in 100 ml of MPC containing 10% fetal calf serum and plated into a 96-well microplate (Falcon #3040) at a cell concentration of 5 x $10^4$ cells/ml as a myeloma cell. At that time, spleen cells and peritoneal cells of mouse BALB/C were added to the above 96-well microplate as a feeder layer at final concentrations of 5 x $10^5$/ml and 1 x $10^5$/ml, respectively. After incubation in a 5% $CO_2$ atmosphere at

37°C, half of the culture medium was replaced every 2 to 3 days by a HAz-ouabain selection medium containing 1 $\mu$g/ml of azaserine (Az), 100 $\mu$M hypoxantine and 0.5 $\mu$M ouabain (Sigma Chemical Company, Saint Louis, Missouri). Three weeks after cell fusion, the supernatant in wells having proliferating cells was subjected to assay of antibody titre against Pseudomonas exotoxin by ELISA (Example 1-(2)). The experimental result is shown in part in Table 5. Fused cells from a well (5G8) with high antibody titre were cultured in a large quantity and were also subjected to cloning using the limiting dilution method. Following culturing for about 2 weeks, anti-Pseudomonas exotoxin antibody titre in the culture supernatant of a cloned cell was examined in the same manner as above. The results are presented in part in Table 6. Finally, two clones 5G8-A8 and 5G8-E11, which stably produce a human IgM antibody against Pseudomonas exotoxin as well as does FK-001, were established.

### Table 5. Anti-Pseudomonas Exotoxin Antibody Titre In Culture Spernatant After Cell Fusion

| Well | Antibody titre $(OD_{405})$ |
|------|------|
| 2C2  | 0.10 |
| 2F3  | 0.11 |
| 2H12 | 0.12 |
| 4G3  | 0.10 |
| 5A8  | 0.26 |
| 5B9  | 0.24 |
| 5G8  | 1.03 |
| 5H7  | 0.19 |
| 5H8  | 0.18 |
| 5H10 | 0.20 |

### Table 6. Anti-Pseudomonas Exotoxin Antibody Titre In Culture Supernatant After Cloning

| Well | Antibody titre ($OD_{405}$) | Well | Antibody titre ($OD_{405}$) |
|------|------|------|------|
| A2   | 0.06 | E3   | 0.04 |
| A4   | 1.25 | E4   | 0.03 |
| A8   | 1.04 | E7   | 0.04 |
| A9   | 0.21 | E11  | 0.79 |
| B11  | 0.99 | F5   | 0.07 |
| C1   | 0.14 | F6   | 0.75 |
| C3   | 0.25 | F7   | 0.49 |
| C5   | 0.08 | F10  | 0.05 |
| D1   | 0.07 | G2   | 0.21 |
| D8   | 0.69 | G4   | 0.80 |
| D9   | 0.08 | H1   | 0.60 |
| E1   | 0.05 | H7   | 0.06 |

#### Example 6

Preparation of antigen-sensitized lymphocytes by in vitro immunization and cell fusion experiment.

Human peripheral blood lymphocytes collected from 25 human volunteers were mixed (2 x $10^6$ cells each) , and 50 ml of RPMI1640 medium (containing 10% of fetal bovine serum) was added thereto. The mixture was incubated for 3 days in 5% $CO_2$ and at 37°C in a culture flask T-75 (Corning #25110). The culture supernatant obtained was mixed with an equal amount of RPMI1640 medium containing 15% of fetal bovine serum, 2 mM of L-glutamine and 1 mM of sodium pyruvate. Human peripheral blood lymphocytes ($10^7$ per ml at the final density) were added to the above mixed medium additionally containing 0.25 to 2.5 µg/ml of pokeweed mitogen (PWM) lectin(P-L Biochemicals, Inc., Milwaukee, WIS), 0.001% of Protein A Sepharose (Pharmacia, Uppsala, Sweden) and 100 ng/ml of inactivated Pseudomonas exotoxin, and 10 ml of the medium was then cultivated for 4 days in 5% $CO_2$ at 37°C in a culture flask T-75 (Corning #25110). After incubation, the antigen-sensitized human peripheral blood was separated by centrifuging and 1 x $10^7$ to 5 x $10^7$ cells thereof were fused with mouse myeloma cells or human lymphoblastoid cells in the same manner as used in preparation of human-mouse hybridoma (Example 3) or human-human hybridoma (Example 4). About two or three weeks later the culture supernatant in the well showing growth was inspected for anti-Pseudomonas exotoxin antibody production, according to ELISA as in Example 1-(2) or RIA as in Example 3-(1). The hybridoma in the antibody producing well was cloned by limiting dilution. About two or three weeks later, the culture supernatant of the cloned cells was similarly inspected for anti-Pseudomonas exotoxin antibody production, and antibody-producing clones were obtained.

#### Example 7

Purification of specific antibody and its characterization.

FK-001 cells transformed by EB virus and established as an anti-Pseudomonas exotoxin antibody-producing cell line were cultivated in the RPMI1640 medium containing 10% of FCS. Its culture supernatant (330 ml ,protein content 750 mg) was subjected to precipitation with 50% saturated ammonium sulfate solution and dialysis against 0.1 M Tris-hydrochloric acid buffer solution (pH8.0) containing 0.2M NaCl. 10 ml of the dialyzate was loaded onto a Sephadex G-200 column (2.5 cm in diameter, 85 cm in length, and 417 ml in volume), and eluted with 0.1M Tris-hydrochloric acid buffer solution (pH8.0) containing 0.2 M NaCl. This gel filtration gave three peaks of protein (IgM, IgG, albumin fractions) and the anti-Pseudomonas exotoxin antibody activity was observed only in the IgM fraction. The recovery rate of protein was 3%, and the specific activity was increased by about eight times. Purified FK-001 antibody was reduced with 2-mercaptoethanol and was subjected to SDS-12.5% acrylamide gel electrophoresis. Two bands of 84 to 86 K (H chain) and 25 to 29 K (L chain) were observed. Taking the data of the gel filtration experiment into account, the apparent molecular weight (M.W.) of FK-001 antibody was estimated to be 950 to 1150 K daltons. Furthermore, purified FK-001 antibody, which was reduced with 2-mercaptoethanol and electrophoresed in SDS-12.5% acrylamide gel, was transferred in a conventional manner to nitrocellulose (Schleicher & Schuell Inc., Keene, NH) and was reacted with alkaline phosphatase-labeled anti-$\mu$, $\gamma$ heavy chain antibody (Kirkegaard & Perry Laboratories, Inc., Gaithersburg, MD) and alkaline phosphatase-labeled anti-$x$, $\lambda$ light chain antibody (E.Y laboratories Inc., San Mateo, CA), respectively. The FK-001 antibody preparation was only reactive with anti-$\mu$ heavy chain and $x$ light chain antibody in the system of so-called Western blotting (Towbin, H et al.: Proc. Natl. Acad. Sci. 76 , 4350, 1979; Burnette W.N.: Anal. Biochem. 112 , 195, 1981). This shows that FK-001 antibody is IgM consisting of a $\mu$ heavy chain and a $x$ light chain.

Example 8

Analysis of antigenic specificity by Western blotting technique.

After SDS/polyacrylamide gel electrophoresis of 3.5 $\mu$g of Pseudomonas exotoxin and 13.2 $\mu$g of molecular weight measuring marker protein (1.5 $\mu$g of phosphorylase b, 1.9 $\mu$g of BSA, 3.3 $\mu$g of egg albumin, 1.9 $\mu$g of carbodehydrogenase, 1.8 $\mu$g of soybean trypsin inhibitor, and 2.8 $\mu$g of $\alpha$-lactoalbumin), the gel was dipped overnight at 4°C in transbuffer (25 mM Tris, 192 mM glycine, pH 8.3, 20% v/v methanol). Then the gel was electrically transferred (30 V, 4.5 h) onto a nitrocellulose membrane (BioRad Laboratories, Richmond, CA) at room temperature, and was blocked by incubation for an hour at room temperature in the 50 mM Tris hydrochloric acid buffer solution, pH 8.0 (TBS), containing 2% of BSA and 0.9% of NaCl. Furthermore, the nitrocellulose membrane was blocked by incubating for an hour at room temperature in the TBS containing 0.1% of BSA and 10% of FCS, and it was further incubated for an hour at 37°C in an aqueous solution of each of (1) No. 1 serum (human serum with anti-Pseudomonas exotoxin antibody activity detectable by ELISA method), (2) culture supernatant of anti-Pseudomonas exotoxin antibody-producing cell lines FK-001, (3) an IgM fraction purified from FK-001 culture supernatant, and (4) culture supernatant of RB virus transformed cell line HI-006, respectively, and then further incubated overnight at 4°C. After being washed five times with TBS (pH 8.0) containing 0.05% of Tween 20, the nitrocellulose membranes were incubated for an hour at 37°C together with the second antibody (peroxidase labeled anti-human immunoglobulin antibody diluted 3,000 times in PBS containing 1% of BSA and 0.05% of Tween 20). Similarly, after the nitrocellulose membrane had been washed five times with TBS containing 0.05% of Tween 20 (pH 8.0), a coloring reagent (TBS containing 0.5 mg/ml of chloronaphthol, 20% of methanol, and 0.03% of $H_2O_2$, pH 7.5) was added to develop the color. As a result, in the lane of the electrophoresis of Pseudomonas exotoxin, in the reaction with No. 1 serum (1), FK-001 culture supernatant (2), and its purified IgM (3), only one band colored in the vicinity of a molecular weight of 68 to 72 K was noted. In the reaction with HI-006 culture supernatant (4), no coloring was observed at all. Meanwhile, in the electrophoresis of a marker protein for measurement of molecular weight, no reaction occurred with any antibody, and no coloring was observed. This shows the specificity of the reaction of FK-001 antibody with the Pseudomonas exotoxin.

EXAMPLE 9

Activity of antibody to neutralize Pseudomonas exotoxin.

Mouse BALB/C 3T3 cells (American Type Culture Collection, Rockville, MD) were suspended in RPMI 1640 medium containing 10% of FCS at $2.0 \times 10^5$ cells/ml, which was then seeded in an amount of 100 $\mu$l per well in a 96-well microplate (Sumitomo Bakelite MS-3096F) and cultivated for 48 hours in 5% $CO_2$ at 37°C. Then, the culture supernatant of EB virus transformed monoclonal antibody-producing cell lines, such as FK-5A5, FK-001, HI-006, was added in an amount of 100 $\mu$l per well. At the same time, Pseudomonas exotoxin solution (10 to 300 ng/ml) was added in an amount of 10 $\mu$l per well. After the exotoxin-treated 3T3 cells had been further cultivated for 24 hours in 5% $CO_2$ at 37°C, the whole medium was replaced with a new medium containing $^3$H-leucine (specific activity 0.2 mCi/mg, 10 $\mu$Ci/ml) and the 3T3 cells were cultivated for 2 hours more. The cells were washed first with PBS then with PBS containing 0.02% of EDTA, and the cells were separated from the plate surface with 0.25% of trypsin solution to prepare a suspension. The cells were incubated at 4°C in 5% trichloroacetic acid (TCA) and washed three times repeatedly until the soluble $^3$H-leucine had been extracted and removed. The portion of $^3$H taken into the TCA-insoluble fraction was separated using a Titertek® Cell Harvester 530, Flow Laboratories and was measured by a liquid scintillation counter (Beckmann Instruments, Inc., Palo Alto, CA). The results are shown in Table 7. Of the anti-Pseudomonas exotoxin antibody-producing cell lines transformed by EB virus, the culture supernatant of FK-5A5 strongly neutralized the cytotoxicity by Pseudomonas exotoxin, while the culture supernatant cf FK-001 showed weak neutralizing activity.

### Table 7. Pseudomonas Exotoxin Neutralizing Activity by Culture Supernatant

| Exotoxin | $^3$H taken into acid insoluble fraction (dpm) | | | |
|---|---|---|---|---|
| | Culture supernatant | | | |
| (ng/ml) | FK-5A5 | FK-001 | HI-006 | 10% FCS-RPMI1640 |
| 0 | 5,372 | 5,171 | 5,160 | 8,290 |
| 0.1 | 6,890 | 4,923 | 3,858 | 4,402 |
| 0.3 | 5,659 | 1,052 | 773 | 714 |
| 1 | 3,498 | 410 | 283 | 182 |
| 3 | 588 | 142 | 135 | 119 |

Example 10

Therapeutic effect of the antibody in mouse experimental Pseudomonas infection.

A Millipore diffusion chamber (Millipore Corporation, Bedford, MA,filter 0.45 $\mu$m pore size, outside diameter 14 mm, inside diameter 10 mm, thickness 2 mm) filled with $10^6$ cells of Pseudomonas aeruginosa PA-103 (exotoxin A-producing strain) was planted into the abdominal cavity of ICR-slc mice (Shizuoka Laboratory Animal Center, Hamamatsu, Shizuoka) (4 to 5 weeks old, male, 5 animals in each group). From the cells growing in the chamber, exotoxin A was stationarily secreted. Immediately after chamber planting, IgM purified from the supernatant of the culture medium of human monoclonal antibody-producing cell line FK-001 (abbreviated as FK-001 antibody purified product),a commercial human immunoglobulin preparation and BSA were intravenously administered, respectively, and the therapeutic effect of each was evaluated in terms of mouse survival over 5 days. The results are shown in Table 8. In the case where 2 $\mu$g of FK-001 antibody purified product was administered to mice, all the animals survived. This therapeutic effect was similar to that achieved when 1 mg of commercial human immunoglobulin preparation was administered to mice.

Table 8.    Therapeutic Effect on Mouse Experimental
           Infection with Pseudomonas aeruginosa

| Antibody | Dose/mouse | Surviving/tested animals |
|---|---|---|
| FK-001 | 2 μg | 5/5 |
|  | 0.4 | 0/5 |
| Commercial | 1,000 | 5/5 |
| immunoglobulin | 200 | 2/5 |
| BSA | 5,000 | 2/5 |

EXAMPLE 11

Diagnostic use of human monoclonal anti-Pseudomonas exotoxin antibody.

Body specimens (body fluid and tissue) such as urine, spinal cord fluid, blood and sputum were isolated from patients suspected to be suffering from a bacterial infection, especially infection with Pseudomonas aeruginosa . The body fluid and tissue possibly containing bacteria were plated onto a 96-well flexible polyvinyl chloride (PVC) microplate (Falcon Microtest III, Beckton Dicknson and Company, Oxnard, CA.) by fixing with formaldehyde as described below. The PVC microplate were treated with 50 μl/well of 5% glutaraldehye in 0.1 M $NaHCO_3$ for 30 min. at 25°C. The PVC microplates were washed 3 times with phosphate buffered saline (PBS) (pH 7.2). The body specimen suspended in PBS was then added and the microplates centrifuged at 500 g for 3 min. The body specimen was fixed by the addition of 200 μl/well of 1% formaldehyde in PBS. After 15 min. at 25°C the microplates were centrifuged again and washed 3 times with PBS. Unbound sites were blocked with 1 % BSA and 1 % polyvinylpyrrolidone in PBS. 50 μl of diluted human monoclonal anti-Pseudomonas exotoxin antibody FK-001 was added to the microplates, which were then incubated for 90 min. at 25°C or overnight at 4°C and washed 10 times with 0.05% Tween 20 [(R)] (polyoxyethylene sorbitan monolaurate; Nakarai Chemicals Ltd., Kyoto, Japan) in PBS. Alkaline phosphatase-conjugated anti-human immunoglobulin M (Kirkegaard & Perry Laboratories, Inc., Gaithersburg, MD) was diluted 1:10 in PBS containing 1 % BSA and 0.05 % Tween 20[(R)] and 50 μl aliquots were added to each well of the microplates. After 2 hrs at 37°C, the microplates were washed 5 times with PBS containing 0.05% Tween 20[(R)]. 100 μl of 3 mg/ml p-nitrophenylphosphoric acid dissolved in 10% diethanolamine buffer (pH 9.1) was added and incubated at 37°C for 30 min. After addition of 20 μl/well 1N NaOH, the optical density at 405 μm was measured by Titertek [(R)] Multiskan automated spectrophotometer (Flow Laboratories Inc., McLean, Virginia).

This procedure permits a diagnosis of whether patients, from whom body specimen is isolated, are infected with exotoxin-producing Pseudomonas aeruginosa , because the optical density of 405 μm will be above the background level and may be relatively high if the blood specimen (body fluid and/or tissue) contains Pseudomonas exotoxin-producing micro-organisms.

**Claims**

1. Synthetic human monoclonal antibody against an exotoxin A of Pseudomonas aeruginosa of which the exotoxin A-neutralizing activity is such as to protect mice from death at a 100 % survival rate at a dose of 2 μg/head in a mouse experimental Pseudomonas aeruginosa infection test.

2. A pharmaceutical composition comprising a carrier or diluent and a pharmaceutically effective amount of a human monoclonal antibody against exotoxin A of Pseudomonas aeruginosa, of which the exotoxin A-neutralizing activity is such as to protect mice from death at a 100 % survival rate at a dose of 2 μg/head in a mouse experimental Pseudomonas infection test.

3. A composition according to claim 2 which further comprises a conventional immunoglobulin preparation.

4. A process for producing a human monoclonal antibody against exotoxin A of Pseudomonas aeruginosa of which the exotoxin A-neutralizing activity is such as to protect mice from death at a 100 % survival rate at a dose of 2 μg/head in a mouse experimental Pseudomonas infection test, which comprises culturing human transformed B lymphocytes which· are established by transforming human B lymphocytes producing the said antibody into immortalized cells capable of permanently proliferating and producing the said antibody by infecting the said B cells with Epstein-Barr virus, and recovering the said monoclonal antibody therefrom.

5. A process for producing a human monoclonal antibody against exotoxin A of Pseudomonas aeruginosa of which the exotoxin A-neutralizing activity is such as to protect mice from death at a 100 % survival rate at a dose of 2 μg/head in a mouse experimental Pseudomonas infection test, which comprises culturing hybridoma cells which are established by fusing Epstein-Barr virus-transformed B lymphocytes producing the said antibody with myeloma cells into immortalized cells capable of permanently proliferating and producing the said antibody and recovering the said monoclonal antibody therefrom.

6. A transformed B lymphocyte capable of permanently proliferating and producing a human monoclonal antibody against exotoxin A of Pseudomonas aeruginosa of which the exotoxin A-neutralizing activity is such as to protect mice from death at a 100 % survival rate at a dose of 2 μg/head in a mouse experimental Pseudomonas infection test, which is produced by transforming human B lymphocyte sensitized to said exotoxin A into immortalized cell capable of permanently proliferating and producing the said antibody by infecting the said B cell with Epstein-Barr virus.

7. A hybridoma cell line capable of permanently proliferating and producing a human monoclonal antibody against exotoxin A of Pseudomonas aeruginosa of which the exotoxin A-neutralizing activity is such as to protect mice from death at a 100 % survival rate at a dose of 2 μg/head in a mouse experimental Pseudomonas infection test, which is produced by fusing human B lymphocyte sensitized to said exotoxin A which has been transformed to permanently proliferate by Epstein-Barr virus infection, with myeloma cells.

**Revendications**

1. Anticorps monoclonal humain de synthèse, actif sur une exotoxine A de Pseudomonas aeruginosa, dont l'activité de neutralisation de l'exotoxine A est telle qu'elle protège la souris de la mort à un pourcentage de survie de 100 %, pour une dose de 2 μg/animal, dans un essai d'infection expérimentale sur la souris par Pseudomonas aeruginosa .

2. Composition pharmaceutique comprenant un véhicule ou un diluant et une quantité pharmaceutiquement efficace d'un anticorps monoclonal humain actif sur une exotoxine A de Pseudomonas aeruginosa , dont l'activité de neutralisation de l'exotoxine A est telle qu'elle protège la souris de la mort à un pourcentage de survie de 100 %, pour une dose de 2 μg/animal dans un essai d'infection expérimentale sur la souris par Pseudomonas.

3. Composition suivant la revendication 2 qui comprend, en outre, une préparation classique d'immunoglobine.

4. Procédé de préparation d'un anticorps monoclonal humain actif sur une exotoxine A de Pseudomonas aeruginosa , dont l'activité de neutralisation de l'exotoxine A est telle qu'elle protège la souris de la mort à un pourcentage de survie de 100 %, pour une dose de 2 μg/animal, dans un essai d'infection expérimentale sur la souris par Pseumodonas, qui consiste à cultiver des lymphocytes B humains transformés, qui sont établis en transformant des lymphocytes B humains produisant l'anticorps en des cellules rendues immortelles et susceptibles de proliférer en permanence et de produire l'anticorps par infection des cellules B par le virus d'Epstein-Barr, et à en recueillir l'anticorps monoclonal.

5. Procédé de préparation d'un anticorps monoclonal humain actif sur une exotoxine A de Pseudomonas aeruginosa , dont l'activité de neutralisation de l'exotoxine A est telle qu'elle protège la souris de la mort à un pourcentage de survie de 100 %, pour une dose de 2 μg/animal, dans un essai d'infection expérimentale sur la souris par Pseumodonas, qui consiste à cultiver des cellules d'hybridome qui sont établies par fusion de lymphocytes B transformés par le virus d'Epstein-Barr produisant cet anticorps, avec des cellules du myélome en des cellules rendues immortelles et susceptibles de proliférer en permanence et de produire l'anticorps, et à en recueillir l'anticorps monoclonal.

6. Lymphocyte B transformé, susceptible de proliférer en permanence et de produire un anticorps monoclonal humain actif sur une exotoxine A de Pseudomonas aeruginosa , dont l'activité de neutralisation de l'exotoxine A est telle qu'elle protège la souris de la mort à un pourcentage de survie de 100 % pour une dose de 2 μg/animal, dans un essai d'infection expérimentale sur la souris par Pseudomonas, qui est préparé en transformant un lymphocyte B humain sensibilisé à l'exotoxine A en une cellule rendue immortelle et susceptible de proliférer en permanence et de produire l'anticorps, par infection de la cellule B par le virus d'Epstein-Barr.

7. Lignée de cellules hybridomes, susceptible de proliférer en permanence et de produire un anticorps monoclonal humain actif sur une exotoxine A de Pseudomonas aeruginosa , dont l'activité de neutralisation de l'exotoxine A est telle qu'elle protège la souris de la mort à un pourcentage de survie de 100 % pour une dose de 2 μg/animal, dans un essai d'infection expérimentale sur la souris par Pseudomonas, qui est préparé par fusion de lymphocyte B humain sensibilisé à l'exotoxine A, qui a été transformé de manière à proliférer de façon permanente par infection par le virus d'Epstein-Barr, avec des cellules du myélome.

## Ansprüche

1. Synthetischer menschlicher monoclonaler Antikörper gegen ein Exotoxin A von Pseudomonas aeruginosa , dessen Exotoxin A-Neutralisierungsaktivität derart ist, daß Mäuse mit einer 100 % Überlebensrate bei einer Dosis von 2 μg/Kopf in einem Pseudomonas aeruginosa -Infektionstest an der Maus vor dem Tod bewahrt werden.

2. Arzneimittel, das einen Träger oder ein Verdünnungsmittel und eine pharmazeutisch wirksame Menge eines menschlichen monoclonalen Antikörpers gegen Exotoxin A von Pseudomonas aeruginosa enthält, dessen Exotoxin A-Neutralisierungsaktivität derart ist, daß Mäuse mit einer 100 % Überlebensrate bei einer Dosis von 2 μg/Kopf in einem Pseudomonas-Infektionstest an der Maus vor dem Tod bewahrt werden.

3. Mittel nach Anspruch 2, das ferner ein übliches Immunoglobulin-Präparat umfaßt.

4. Verfahren zur Herstellung eines menschlichen monoclonalen Antikörpers gegen Exotoxin A von Pseudomonas aerugi nosa , dessen Exotoxin A-Neutralisierungsaktivität derart ist, daß Mäuse mit einer 100 % Überlebensrate bei einer Dosis von 2 μg/Kopf in einem Pseudomonas-Infektionstest an der Maus vor dem Tod bewahrt werden, das die Kultivierung von menschlichen transformierten B-Lymphocyten, die durch Transformation menschlicher, den Antikörper produzierender B - Lymphocyten in unsterbliche Zellen geschaffen werden, die zur permanenten Proliferation und Herstellung des Antikörpers durch Infektion der B-Zellen mit Epstein-Barr -Virus befähigt sind, und die Gewinnung des monoclonalen Antikörpers davon umfaßt.

5. Verfahren zur Herstellung eines menschlichen monoclonalen Antikörpers gegen Exotoxin A von Pseudomonas aerugi nosa , dessen Exotoxin A-Neutralisierungsaktivität derart ist, daß Mäuse mit einer 100 % Überlebensrate bei einer Dosis von 2 μg/Kopf in einem Pseudomonas-Infektionstests an der Maus vor dem Tod bewahrt werden, das die Kultivierung von Hybridomazellen, die durch Fusion Antikörper-produzierender, Epstein-Barr-Virus-transformierter B-Lymphocyten mit Myelomazellen in unsterbliche Zellen geschaffen werden, die zur permanenten Proliferation und Herstellung des Antikörpers befähigt sind, und die Gewinnung des monoclonalen Antikörpers davon umfaßt.

6. Transformierter B Lymphocyt, der zur permanenten Proliferation und Herstellung eines menschlichen monoclonalen Antikörpers gegen Exotoxin A von Pseudomonas aeruginosa befähigt ist, dessen Exotoxin A-Neutralisierungsaktivität derart ist, daß Mäuse mit einer 100 % Überlebensrate bei einer Dosis von 2 µg/Kopf in einem Pseudomonas-Infektionstest an der Maus vor dem Tod bewahrt werden, der durch Transformation eines menschlichen, gegen Exotoxin A sensitivierten B -Lymphocyten in eine unsterbliche Zelle, die zur permanenten Proliferation und Herstellung des Antikörpers durch Infektion der B-Zelle mit Epstein-Barr-Virus befähigt ist, hergestellt wird.

7. Hybridoma-Zellinie, die zur permanenten Proliferation und Herstellung eines menschlichen monoclonalen Antikörpers gegen Exotoxin A von Pseudomonas aeruginosa befähigt ist, dessen Exotoxin A-Neutralisierungsaktivität derart ist, daß Mäuse mit einer 100 % Überlebensrate bei einer Dosis von 2 µg/Kopf in einem Pseudomonas-Infektionstests an der Maus vor dem Tod bewahrt werden, die durch Fusion eines menschlichen, gegen Exotoxin A sensi tivierten B-Lymphocyten, der durch Epstein-Barr-Virusinfektion zur permanenten Proliferation transformiert worden ist, mit Myelomazellen hergestellt wird.